# EUROPEAN PATENT APPLICATION

(11) **EP 4 035 703 A1**
(43) Date of publication of application: **03.08.2022**
(21) Application number: 21154249.3
(22) Date of filing: 29.01.2021
(51) Int. Cl.: A61M 1/16, A61M 1/28

(54) **PREPARATOR SYSTEM**

(71) Applicant: Bellco S.r.l., 41037 Mirandola (IT)
(72) Inventor: MARRA, Antonio Giuseppe, 41037 Modena (IT); VERATTI, Andrea, 41037 Modena (IT)
(74) Representative: Maschio, Antonio

(57) **Abstract**

A dialysis system comprising a preparator and cycler, wherein said preparator comprises a purified water source fluidly connectable to at least one container containing one or more solutes for dissolution, at least one measuring device, a gyroscope configured to measure the orientation and/or angular velocity of the preparator or a component thereof and an accelerometer configured to measure linear acceleration of the preparator or a component thereof.

## Description

### FIELD

This invention relates to a preparator system for use in dialysis. In particular, a preparator which is configured to detect shock and/or to detect the need for calibration in any necessary measurement device is described herein.

### BACKGROUND

The dialysis process aims to remove waste products and excess fluid from the blood of a patient with a disease such as renal failure. Dialysis might be required regularly (e.g., daily) for an extended period of time or, in some cases, life. Therefore, to minimise disruption to the life of the patient, dialysis is often self-administered using a dialysis system set up in the home of the patient.

Generally, a patient needing to complete dialysis at home is provided with a catheter that includes a transfer set for selective connection to a dialysis solution or a drain bag. In peritoneal dialysis solution ('dialysate') comprising water with other electrolytes, and including an osmotic agent (generally dextrose) for peritoneal dialysis, is warmed to body temperature and then introduced into the patient via the catheter. The solution remains here for a period of time required to clean the blood of waste products, toxins and excess fluid and these are then removed from the body to leave the right amounts of electrolytes and nutrients in the blood. A cycler machine may repeat the exchange process a number of times as required for a particular patient. In hemodialysis, the blood of the patient is circulated through a dialyser, which is comes into contact with dialysate and metabolites pass from the blood into the dialysate through osmotic action.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. For dialysis to function correctly and safely, it is very important that the concentration of solutes in the dialysate is accurate.

The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This reconstitution of the components occurs in a preparator which contains various measuring devices, including scales to measure the dialysate components by mass.

Environmental noise and vibrations of any kind may affect the preparator. The scales of the preparator must be very sensitive in order to function correctly and accurately, to produce a dialysate comprising the correct solute concentrations for the patient concerned. The scales commonly need to be calibrated every time the preparator is moved. As the home may be more noisy than a hospital environment, and the dialysis system may be more likely to be moved, knocked and transported in the home, the need remains for a preparator which remains accurate in this environment, while still being easy to use.

### SUMMARY

A first aspect of the present invention provides a dialysis system comprising a dialysis preparator and cycler, the preparator comprising a purified water source fluidly connectable to at least one container containing one or more solutes for dissolution or one or more concentrates in liquid form for dilution, at least one measuring device, a gyroscope and an accelerometer. Dissolution of the solutes forms a dialysate fluid for delivery to the cycler and then for delivery to a patient or cycling in a dialysis machine. In embodiments the system further comprises a controller.

In embodiments, a hydraulic flow generator is provided in the dialysis system in at least one of the fluid paths between the water source and the at least one container and the at least one container and the cycler.

In embodiments the preparator comprises a purified water source fluidly connectable to a plurality of containers each containing one or more solutes.

In embodiments, said dialysis system is suitable for peritoneal dialysis, hemodialysis, hemofiltration, hemodiafiltration or ultrafiltration. In embodiments, said dialysis system is for suitable peritoneal dialysis.

A second aspect of the present invention provides a preparator for use in a dialysis system. The preparator comprises a purified water source fluidly connectable to at least one container containing one or more solutes for dissolution, at least one measuring device, a gyroscope and an accelerometer.

In embodiments the at least one measuring device is scales for measuring mass, specifcially the mass of the solutes and/or water and/or fluid within the preparator. Additional measuring devices may be included. Additional measuring devices may include a thermometer for measuring the temperature of the fluid within the preparator.

The at least one measuring device needs to be calibrated. Calibration can be performed as need, for example before use, and during use if the preparator is subject to shock or movement which may disrupt the measuring device. The calibration may be carried out automatically on receiving feedback that the preparator has been moved from the gyroscope and/or accelerometer. The calibration may be carried out electronically. Disruption of the measuring device can be considered likely if the shock or movement records a change in acceleration and/or orientation and/or velocity outside of a pre-determined range.

In embodiments, the gyroscope measures the orientation and velocity, particularly angular velocity, of the preparator or any component thereof, but in particular the at least one measuring device. If the gyroscope measures a change in the orientation and/or velocity of the preparator or any component thereof, in embodiments it provides feedback. Preferably this feedback is sent to a controller. Alternatively, this feedback may be sent directly to the at least one measuring device, or alert a user.

In embodiments, the accelerometer measures measuring linear acceleration of the preparator of any component thereof, but in particular the at least one measuring device. If the accelerometer measures a change in the acceleration of the preparator or any component thereof, in embodiments it provides feedback. Preferably this feedback is sent to a controller. Alternatively, this feedback may be sent directly to the at least one measuring device, or alert a user.

In embodiments, feedback from the gyroscope and/or feedback from the accelerometer indicating a change in acceleration and/or orientation and/or velocity (preferably angular velocity) of the preparator or any component thereof causes the at least one measuring device to be re-calibrated, and/or recent measurements to be cancelled and re-taken by the measuring device and/or the alert the user to incorrect use of the dialysis system, and/or stop the dialysis system from working if it is being used incorrectly.

In embodiments the preparator includes a controller. The controller may be a computer. The computer may respond to feedback from the gyroscope and/or feedback from the accelerometer, and may be integrated or remotely configured.

The preparator described herein can be used in any dialysis system, but preferably is suitable for use in the dialysis system described herein.

Preferably the dialysis system and preparator described herein is suitable for use outside a medical facility, such as at home.

A third aspect of the present invention provides a method of use of a dialysis system as described herein, and specifcially the preparator as described herein.

A fourth aspect of the present invention provides a kit for use in a dialysis system.

### BRIEF DESCRIPTION OF THE DRAWINGS

Various examples of the invention will now be described, by way of illustration only, with reference to the figures.
**Figure 1** is a schematic drawing illustrating an automated peritoneal dialysis machine according to the prior art.
**Figure 2** is a block diagram of an automated peritoneal dialysis preparator and cycler machine according to the present invention.

### DETAILED DESCRIPTION

Unless defined otherwise, all technical and scientific terms used generally have the same meaning as commonly understood by one of ordinary skill in the art.

The articles "a" and "an" are used to refer to one or to over one (*i.e.,* to at least one) of the grammatical object of the article. For example, "an element" means one element or over one element.

The term "and/or" as used herein means and, or alternatively. That is, the features referred to may occur independently or together.

The term "accelerometer" as used herein refers to a device used for measuring linear acceleration. The accelerometer may be any type of accelerometer, such as a piezoelectric accelerometer, a piezoresistance accelerometer or a capacitive accelerometer or MEMS. The accelerometer may be electronically controlled and maintained, and give measurements via electronic means, such as a display. An accelerometer may also measure a change in acceleration, including deceleration.

The term "calibrate" as used herein refers to the documented comparison of a measurement device against a traceable reference device. Specifically, the measuring devices described herein are calibrated at least before use, and during use if movement of the device or the preparator in which the device situated is recorded. To "re-calibrate" means to calibrate again, after a movement or other change to the device.

The term "controller" as used herein refers to a device which regulates a system or apparatus. Commonly the controller is a computer or computational device. The controller may provide information to a user.

The term "comprising" includes, but is not limited to, whatever follows the word "comprising". Use of the term indicates the listed elements are required or mandatory but that other elements are optional and may be present.

The term "component" as used herein refers to any part or element of a larger whole.

The term "consisting of" includes and is limited to whatever follows the phrase "consisting of". The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid diffuse through a membrane (in the case of peritoneal dialysis, through the peritoneum of the patient) during dialysis. An initial dialysate used for therapy typically contains electrolytes close in concentration to the physiological concentration of electrolytes found in blood. However, the concentration of the dialysate can change over the course of therapy, and can further be adjusted as desired. It is important that the dialysate is maintained at the correct concentration for solutes or the patient. Dialysate is generated through the use of a preparator and may be stored in a tank within the dialysis machine.

The term "dialysis" refers to the process of removing excess water, solutes, and toxins from the blood in people whose kidneys can no longer perform these functions naturally. Different types of dialysis include hemodialysis, peritoneal dialysis, hemofiltration, hemodiafiltration or ultrafiltration. In hemodialysis, blood is pumped out of the body of the patient and filtered by passing it under high pressure usually through a bundle of hollow fibres in a filter called a dialyzer. Dialysate passes through the dialyzer in the opposite direction to the blood, outside of the fibres. Low molecular weight metabolites, such as urea, pass from the blood into the dialysate through osmotic action, and thus the dialyzer acts as an artificial kidney before the blood is returned to the patient by tubes connecting the patient to the machine. In peritoneal dialysis, dialysate flows through a catheter into part of the patient's abdomen. The lining of the abdomen (peritoneum) acts as a filter and removes waste products from the blood. After a set period of time, the fluid with the filtered waste products is removed from the abdomen and discarded.

The term "dialysis machine" (sometimes simply referred to as a "machine") as used herein refers to any machine capable of performing dialysis, but in particular a machine containing a dialyzer for hemodialysis or a machine suitable for delivering peritoneal dialysis fluid to a patient. Usually a dialysis machine will comprise at least a preparator and some kind of pump or cycler, fluidly connected to each other and fluidly connectable to a patient.

The term "dialysis system" as used herein refers to any system comprising components for use in any form of dialysis, including hemodialysis, peritoneal dialysis, hemofiltration, hemodiafiltration or ultrafiltration.

The term "dialysis flow path" refers to a fluid pathway or passageway configured to convey a fluid, such as dialysate and/or blood, wherein said pathway forms at least part of, preferably the whole of, a fluid circuit for peritoneal dialysis, hemodialysis, hemofiltration, hemodiafiltration or ultrafiltration. A preparator may be included in the flow path. A dialysis machine may be included within the flow path. A dialyzer may be included in the flow path.

The term "feedback" or "feed back" as used herein refers to reporting information and in particular reporting a change in information. It is envisaged that the accelerometer and/or gyroscope described herein will provide feedback to the at least one measuring device if the preparator has been moved in any way or suffered a shock, so that the measuring device is re-calibrated. The feedback may be sent to a controller which controls the calibration process and/or the functioning of the preparator and/or the functioning of the dialysis system. The feedback could be in the form of a screen display and/or a visual or audible alarm. The feedback may constitute instructions, for example instructions to re-calibrate the at least one measuring device.

The term "fluid" can be any substance without a fixed shape that yields easily to external pressure such as a gas or a liquid. Specifically, the fluid can be water, preferably purified water, and can be water containing any solutes at any concentration. The fluid can be used as a solvent (to dissolve one or more solutes). The fluid can also be dialysate of any type including fresh, partially used, or spent. The fluid can also contain one or more dissolved gas. The fluid may be blood.

The term "fluidly connectable" refers to the ability to provide passage of fluid from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Most preferably the connections are provided by tubes or pipes.

The term "fluidly connected" refers to a particular state or configuration of one or more components such that fluid can flow from one point to another point. The connection state can also include an optional unconnected state or configuration, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "formed" or "formed of" as used herein refers to a material comprising any of the materials, elements, composited or compounds listed.

The term "gyroscope" as used herein refers to a device used for measuring or maintaining orientation and angular velocity. Angular velocity is the change in rotational angle per unit of time. Angular velocity is expressed in deg/s (degrees per second). The gyroscope may be any type of gyroscope, such as a rotary gyroscope, a vibrating structure gyroscope or an optical gyroscopes. The gyroscope may be electronically controlled and maintained, and give measurements via electronic means, such as a display. When the gyroscope records as change in orientation and/or angular velocity, it may provide feedback. Wherein the gyroscope is referred to herein as measuring "velocity", this refers to angular velocity.

The term "inlet" can refer to a portion of a component through which fluid and/or gas can be drawn into a component, conduit, or fluid passageway of any type, such as a bag, container, conduit, tube or pipe of any type.

The term "measuring" or "to measure" can refer to determining any parameter or variable. The parameter or variable can relate to any state or value of a system, component, fluid, gas, or mixtures of one or more gases or fluids. The term "monitor" refers to continuous measurements.

The term "measurement" refers to data or information of the parameter or variable determined.

The term "measurement device" as used herein refers to any device for carrying out a measurement. A measurement device may include for example scales for measuring mass, or a thermometer for measuring temperature.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The term "noise" as used herein refers to any vibrational movement, and in particular audible vibrations, in the environment.

The term "nozzle" as used herein refers to a fitting, usually at the end of a tube or pipe, which controls the flow of a fluid (liquid or gas) through said fitting. It may control (in speed, direction or otherwise) the entry of the fluid into a container, especially a bag. It may accelerate or decelerate the fluid. It may control the direction of the fluid. The nozzle may control the density of spray of the fluid and direction of spray of fluid from the nozzle.

The term "outlet" refers to a portion of a component through which fluid or gas can be pulled out of said component and into another component, conduit, or fluid passageway of any type.

The term "pass through" refers to an action of fluid or gas passing a component positioned in a flow path. The fluid or gas can enter an inlet of the component and exit via an outlet of the component to continue the flow path.

The term "preparator" as used herein refers to a machine in which dialysate is prepared for dialysis. A preparator comprises a purified water source fluidly connectable to at least one container containing one or more solutes for dissolution and at least one measuring device. The preparator dissolves the solutes. Preferably each at least one container is fluidly connectable to a chamber for receiving a solution of the solutes for preparation of a dialysate, said chamber being fluidly connectable to the cycler. The dialysate must be prepared to a recipe specific to the patient. Once prepared, dialysate can be used immediately or stored in a tank within the preparator. The preparator described herein comprises multiple components, including at least one measuring device, an accelerometer and a gyroscope.

The term "pressure" refers to a force exerted by a gas or liquid on the walls of a component, container, or conduit.

"Purified water" can be defined as water produced by distillation, deionization, reverse osmosis, or other suitable processes that remove impurities in water.

The term "reconstituting" or "re-constituting as used herein refers to restore to an original state. In the context of the solutions of the invention, this term is used to mean returning a solution to its original form, before liquids were removed, or in creating a solution from concentrated liquid and/or dried components.

The term "reconstituted" or "re-constituted" refers to an item, product or solution produced after reconstituting as defined above.

The term "scale" or "scales" as used herein refers to a device for measuring the weight or mass of a substance, whether the substance may be liquid of solid. The scale may be mechanical or electronic. The scale may provide feedback to a controller and/or a user. The scale may be calibrated, electronically or manually. A scale may function in a system of multiple scales.

The term "shock" refers to movement or vibration of a device described herein, such as being transported, dropped, hit or a change in direction and/or velocity and/or angular velocity, and/or orientation and/or acceleration, especially if the change is rapid.

The term "solute" as used herein refers to a substance dissolved in a solution. The solute may comprise one or more chemicals or compounds. Solutes as used herein, before being dissolved in a solvent, may exist as entirely or partially dried, gels, concentrated liquids, pellets or powders, or any combination of these forms.

The term "solution" as used herein refers to a liquid mixture in which a solute is uniformly distributed within a solvent. A "solution for dialysis" may refer to any solution which can be used in any form of dialysis. It is envisaged that the solutions described herein contain specific concentrations of solutes.

The term "solvent" as used herein refers to a substance which dissolves a solute. Preferably the solvent is a liquid, and most preferably it is water.

As used herein, the "Venturi effect" reduction in fluid pressure, and increase in velocity, caused by a fluid flowing through a constricted section of a tube or pipe. Preferably the constriction is caused by a reduction in diameter of the tube or pipe.

The term "water purification unit" refers to any single component or system which can produce purified water (as defined above) from tap water or other water containing any type of impurity.

### System and apparatus

The present invention relates to an improved dialysis system (10) that includes a preparator and cycler for *in situ* preparation of dialysate for delivery and drainage from a patient.

The present invention includes a preparator for use in a dialysis system wherein said preparator comprises a purified water source fluidly connectable to at least one container containing one or more solutes for dissolution, at least one measuring device, a gyroscope and an accelerometer. In embodiments, the system also comprises a controller.

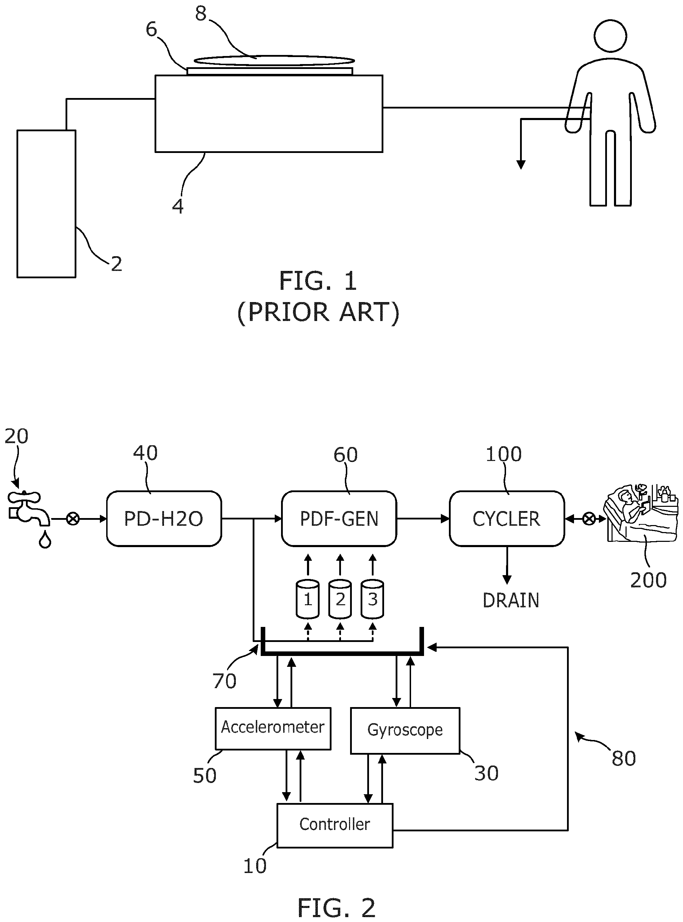

The basic concept of a type of dialysis machine according to the prior art is shown in FIG. 1 of the accompanying drawings. This figure illustrates peritoneal dialysis specifcially, but the system described herein can be used in any type of dialysis, such as hemodialysis, hemofiltration, hemodiafiltration or ultrafiltration. In figure 1, a machine is fluidly connected to a patient via a catheter and transfer set and the machine has a container or bag 2 with a fresh solution supply of dialysate which is delivered to a heating bag 8 placed on a heat source, such as a heating plate 6 to warm the dialysate prior to delivery of this fluid to the patient. The fluid remains in the patient for a predetermined dwell time and then removed from the patient, normally being collected in a drainage bag. The parts are fluidly connectable to provide fluid paths through the system creating a fluid system which is controlled by a controller 4. The machine includes a pump for transport of the fluid to and away from the patient, valves to control the fluid flows, a heat source to warm the dialysate prior to its introduction into the patient and sensors connected to a controller to monitor and control the process.

The machine of FIG. 1 controls and monitors the introduction of fresh peritoneal dialysis fluid (dialysate, or PDF) into the peritoneal cavity via the inlet and transfer set and the elimination of waste fluid via the transfer set and outlet. Sequential treatment cycles are carried out on a patient, normally overnight.

The machine controls and monitors the introduction of fresh dialysate into the abdominal cavity via the inlet and transfer set and the elimination of waste fluid via the transfer set and outlet. Sequential treatment cycles are carried out on a patient, normally overnight. However, the production of sufficient dialysate for these cycles, particularly in a home-based setting, can be problematic.

The content of the dialysate is specific to the patient. For the dialysis to work correctly, the contents of the dialysate must be correct. Dialysate is a solution of pure water, electrolytes and salts, such as bicarbonate and sodium, and osmotic agents such as dextrose in the case of peritoneal dialysis. An initial dialysate used for therapy typically contains electrolytes very similar in concentration to the physiological concentration of electrolytes found in blood. The purpose of dialysate is to pull toxins from the blood into the dialysate via diffusion. This means that the concentration of the dialysate can change over the course of therapy and may need to be adjusted during dialysis. In addition, the content of the dialysate may need to be varied depending upon to condition of the patient.

In a situation wherein dialysis is caried out at home, the dialysis machine must be able to reconstitute powders (dissolve them in a liquid), or dilute liquid concentrates, and proportion them to create a fluid suitable for delivery to a patient. This occurs in the preparator in the dialysis machine. Ideally, bags are provided that are equipped with nozzles to generate a turbulent flow, particularly using the Venturi effect, necessary to dissolve the powder and a hydraulic machine generates the flow to move different amounts of fluid. The preparator has a reconstitution section wherein the powders are dissolved and a proportioning part to proportion the right amount of concentrate formed from the dissolved powders.

The reconstitution part of the system includes the bags filled with the powders required to form the correct concentrate, such as dextrose, magnesium and calcium and lactate bicarbonate and sodium chloride. Electro valves are provided on entry and exit to the bags to deviate the flow path. A flow is required to ensure that the powders are completely dissolved, and a heater may be provided to aid dissolution and to raise the temperature of the resultant dialysate . The proportioning part of the system requires a more accurate flow rate adjuster, such as a peristaltic pump, to move precise amounts of liquid in order to proportion the correct amount of concentrate, together with a scale or scale system to measure the mass of the solutions in order to proportion the right amount of concentrate.

One challenge with this system is to ensure that the scales and any other measurement device in the preparator, such as the pump encoder or flow meter, remain accurate. Particularly in the home environment, the dialysis machine is likely to be subject to increased noise and vibrations, and possible shock. This may be caused by the necessary moving or accidental manhandling of the dialysis machine or any component of the dialysis system, especially the preparator. Such movements are likely to disrupt any measuring devices in the preparator, and in particularly the scales are these need to be highly accurate and highly sensitive to function correctly. Furthermore, if the machine is subject to shock, incorrect measurements may be made. It is also possible that the machine may be used incorrectly, such as being places on a surface which in not flat, in a way which affects the measuring devices in the preparator.

A first aspect of the present invention provides a dialysis system comprising a dialysis preparator and cycler, the preparator comprising a purified water source fluidly connectable to at least one container containing one or more solutes for dissolution at least one measuring device, a gyroscope and an accelerometer. Dissolution of the solutes forms a dialysate fluid for delivery to the cycler and then for delivery to and drainage from a patient.

Figure 2 provides a high level block diagram of the preparator and cycler. The system delivers water, such as purified drinking water 40 from a tap 20, into one or more containers 60 with different powders to create a concentrate and then moves this concentrate to a mixing bag to create the peritoneal dialysis fluid. Drinking water is supplied to a purification system and this purified water is then moved through powdered chemical constituents to create dissolution of the chemicals and proportioned to obtain the PDF solution for passing to the cycler 100. The powder concentrates may be provided in compartmentalized constituents bag with each compartment having an inlet and outlet with a two-way valve whereby purified water can enter each compartment and is then delivered to a mixing bag prior to delivery to the cycler. The cycler then delivers fresh dialysate (PDF in the case of peritoneal dialysis as exemplified here) to the patient 200 and removes waste fluid via the drain outlet.

Scales 70 [VA1]monitor the mass of the containers 60. The accelerometer 50 and gyroscope 30 measure the acceleration and/or orientation and/or velocity of the preparator or any component thereof, but in particular of the scales 70 or any other measuring device, and provide feedback on any change to the controller 10. If accelerometer 50 and, or alternatively, the gyroscope 30 detect a change above a predetermined threshold in the orientation and/or velocity and/or acceleration of the preparator or any component thereof, in particular the scales, this prompts the controller 10 to instruct the scales 70 to re-calibrate via bidirectional connection 80.

The accelerometer 50 and gyroscope 30 may monitor the acceleration and/or orientation and/or velocity of the preparator or any component thereof, but in particular of the scales 70, or any other measuring device, continuously during use or periodically. They may provide feedback, to the controller and/or directly to the scales or any other measuring device continually or periodically, or only wherein a change the acceleration and/or orientation and/or velocity outside of a pre-determined range is recorded.

It is envisaged that the accelerometer and gyroscope would have additional connections to the body of the preparator, and optionally to the casing of the dialysis machine. Therefore, they could monitor and report to the controller any change in the orientation and velocity across the entire dialysis machine, and initiate re-calibration of the scales, and any other measuring devices, if any part of the dialysis machine is subject to movement, acceleration, vibration or shock.

The accelerometer and/or the gyroscope may directly feedback to the scales (or any measuring device) that movement, vibration, or shock has occurred or has changed, and the scales should be re-calibrated. This would enable the preparator to function without the controller. Preferably re-calibration occurs if the change in orientation and/or velocity and/or acceleration detected by the accelerometer and/or the gyroscope falls outside of a pre-determined range.

It is envisaged that the accelerometer and/or the gyroscope may monitor the acceleration and/or orientation and/or velocity of the entire dialysis system, but in preferred embodiments their sensitivity is set as such the monitor the acceleration and/or orientation and/or velocity of the preparator or any component thereof

A series of accelerometers and/or gyroscopes could be used to monitor a change in the orientation and/or velocity and/or acceleration of different parts of the system and/or of multiple measuring devices within the system.

For example, since the scale used in the preparator is highly accurate, this topology of the preparator is very sensitive and affected by shocks and displacements, as well as by the inclination of the floor. If the equipment is moved, the gyroscope can detect the new position and perform a new calibration using the data come from this sensor. The gyroscope can be used to determine the correct position of the machine and detect if the machine has been moved.

The use of the accelerometer permits determination of whether the equipment has been moved by patient introducing a weight error in a particular phase. This avoids errors in weighing of components in the preparator.

The controller may have a display, and display change in the orientation and/or velocity and/or acceleration of different parts of the system. It may display and record any re-calibration which occurs, or ask a user to perform a manual claibration. The controller may also issue an audible or visual alarm if the accelerometer and gyroscope determine that the device is being used incorrectly. That is, if the dialysis machine is placed on a non-horizonal or uneven surface such that the scales and/or other measuring devices cannot be calibrated correctly or cannot work correctly. The controller may also shut down the dialysis machine or entire dialysis system if incorrect use is detected, or deny user access to the treatment. Sut down for incorrect use may occur if a measuring device records a value immediately after the accelerometer and, or alternatively, the gyroscope has recorded a change in orientation and/or velocity and/or acceleration of any part of the dialysis machine or preparator, but particularly a change in orientation and/or velocity and/or acceleration of said measuring device, the controller detects this, re-calibrates the measuring device and re-takes the measurement. The incorrect measurement taken after the shock is cancelled. This prevents incorrect measurements being made if the machine is knocked, moved, or dropped during use.

In embodiments, a hydraulic flow generator is provided in the dialysis system in at least one of the fluid paths between the water source and the at least one container and the at least one container and the cycler. Preferably the at least hydraulic flow generator is a variable flow generator providing an operative flow range of 50-400ml/min. In preferred embodiment of the system described herein, one or a plurality of hydraulic flow or pressure generators is provided throughout the fluid paths to optimize dissolution of the solutes, mixing of the solution and/or cycling of the dialysate thus formed.

It is to be appreciated that all parts of the system should be leak-proof so that fluids cannot escape. The container or bags of the system described herein may be made of any material, preferably a polymer, most preferably a plastic. The bag may be rigid or flexible. Preferably the bag is flexible, resistant and stretchable, most preferably in any direction. The bag may be made of a multi-layered material, or a single layered material.

Each container of the preparator described herein is fluidly connectable to a chamber for receiving a solution of the solutes for preparation of the dialysate, said chamber being fluidly connectable to the cycler (see FIG.2). Preferably at least one hydraulic flow generator is provided in a fluid path between the containers and the chamber and/or the chamber and the cycler.

The system preferably uses a bag containing a v-shaped, funnel shaped or cone shaped section, with the opening for the nozzle at the point of the cone. Such a shape maximises the mixing of the fluid spray leaving the nozzle with the solutes in the bag to aid dissolution of the solutes therein. However, the bag can be replaced by a solid container, preferably shaped with a cone-shaped part in which to fit a nozzle. Said container may be a box or carton, and is preferably made of a plastic.

Examples of solutes for dissolution to form the dialysate may for example comprise the following or any combination thereof:- all electrolytes, powdered acids and bases, citric acid, sodium chloride, hydrogen carbonate, calcium and magnesium ions and salts, sodium, potassium, citric acid, sodium bicarbonate and all bicarbonate salt forms, sodium lactate, and osmotic agents such as glucose, dextrose, polydextrose, polydextrine and xylitol.

The dialysis system described herein is suitable for peritoneal dialysis, hemodialysis, hemofiltration, hemodiafiltration or ultrafiltration. In preferred embodiments, said dialysis system is for suitable peritoneal dialysis.

A second aspect of the present invention provides a preparator for use in a dialysis system. The preparator comprises a purified water source fluidly connectable to at least one container containing one or more solutes for dissolution, at least one measuring device, a gyroscope and an accelerometer.

The preparator is as described above and as shown in FIG. 2.

The at least one measuring device comprises scales or a system of multiple scales, for measuring mass, specifcially the mass of the solutes and/or water and/or fluid within the preparator. The scales measure the mass of the solutions in the preparators order to proportion the right amount of concentrate. The scales may be electronic, automatic or manual.

The at least one measuring device needs to be calibrated. Calibration needs to be performed at least before every use, and during use if the preparator is subject to shock or movement which may disrupt the measuring device. The calibration may be carried out automatically on receiving feedback that the preparator has been moved from the gyroscope and/or accelerometer. The calibration may be carried out electronically. The controller may carry out or initiate calibration. Calibration may be carried out by a device, such as a computing device, attached to the measuring device.

Calibration may be based on a normal range which is pre-determined, and any change in orientation and/or velocity and/or acceleration outside of this pre-determined range may cause the at least one measuring device to be re-calibrated. This means that re-calibration will not occur constantly due to very tiny vibrations (such as normal speaking). The pre-determined normal range is dependent on how sensitive the at least one measuring device is. If a very large or long lasting (more and 1,2 ,3, 5, 10 or 20 seconds) change in orientation and/or velocity and/or acceleration (that is, a sudden physical shock) of the dialysis system, preparator or any component thereof is detected, the dialysis process may be halted. This may also cause recent measurements of the at least one measuring device to be cancelled. The controller may cause the dialysis machine to stop and may indicate this to the user. Indication may be via a display and/or an audible or visual alarm. Alternatively feedback from the gyroscope and/or feedback from the accelerometer directly may halt or shut down the entire dialysis system, or just the dialysis machine in this situation, especially in the case wherein the gyroscope and/or feedback from the accelerometer record a large shock to any part of the dialysis system, especially to the at least one measuring device. There may be more than one pre-determined range. For example, if the change in orientation and/or velocity and/or acceleration falls outside of a first pre-determined range but within a second pre-determined range this may cause the at least one measuring device to be re-calibrated. If the change in orientation and/or velocity and/or acceleration falls outside of the first and second pre-determined ranges, the dialysis machine may shut down due to shock and/or cancel the most recent measurements. In other words, the pre-determined ranges may be staggered and encompass different levels of shock.

Additional measuring devices may be included. Additional measuring devices may include a pH monitor for measuring the pH of the dialysate, and a thermometer for measuring the temperature of the fluid within the preparator, a flow meter and an encoder.

The gyroscope of the preparator measures the orientation and angular velocity of the preparator. If the gyroscope measures a change in the orientation and angular velocity of the preparator, in embodiments it provides feedback. Preferably this feedback is sent to a controller. Alternatively, this feedback may be sent directly to the at least one measuring device, or alert a user. Alerts may be in the form of a visual or audible display to the user, preferably from the controller.

The accelerometer of the preparator measures measuring linear acceleration of the preparator. If the accelerometer measures a change in the acceleration of the preparator, in embodiments it provides feedback. Preferably this feedback is sent to a controller. Alternatively, this feedback may be sent directly to the at least one measuring device, or alert a user. Alerts may be in the form of a visual or audible display to the user, preferably from the controller.

The preparator described herein can be used in any dialysis system, but preferably is suitable for use in the dialysis system described herein.

Preferably the dialysis system and preparator described herein is suitable for use outside a medical facility, such as at home.

The system described herein is particularly suitable for use in dialysis carried out at home, or another environment away from a hospital or medical practice. They may also be used in a hospital or any kind of medical practice.

### Methods

A third aspect of the present invention provides a method of use of a dialysis system as described herein, and specifcially a method of use of a preparator as described herein.

A method of re-constituting a solution comprising one or more solutes in a fluid is described herein. Said solution is a dialysate. Said method comprises the steps of:-
flowing a fluid, preferably a purified water, through a preparator, said preparator comprising a at least one container fluidly connected to the purified water source, said container containing one or more solutes for dissolution, and said preparator further comprising at least one measuring device, a gyroscope and an accelerometer;
wherein the at least one measuring device is a scale or scale system;
wherein said scale or scale system measures the mass of the solutions created by the water flowing into the at least one container in order to create a dialysate comprising the correct concentration of solutes;
wherein the gyroscope is configured to measure the orientation and/or angular velocity of the preparator or a component thereof and the accelerometer is configured to measure linear acceleration of the preparator or a component thereof;
wherein if the gyroscope measures a change in the orientation and/or angular velocity of the preparator or a component thereof, it provides feedback which causes the at least one measuring device to be re-calibrated if the change in orientation and/or angular velocity of the preparator or a component thereof falls outside of a pre-determined range; and/or
wherein if the accelerometer measures a change in the linear acceleration of the preparator or a component thereof, it provides feedback which causes the at least one measuring device to be re-calibrated if the change in linear acceleration of the preparator or a component thereof falls outside of a pre-determined range; and/or
wherein if the accelerometer measures a change in the linear acceleration of the preparator or a component thereof falling outside of a pre-determined range, and/or the gyroscope measures a change in the orientation and/or angular velocity of the preparator or a component thereof outside of a second pre-determined range the dialysis system is shut down due to incorrect use.

The at least one measuring device may be a scale or scale system.

Additional steps may include one or more of the following:-
Heating the fluid to an appropriate temperature to improve dissolution before said fluid enters the preparator;
Controlling the fluid flow rate into the preparator;
Selecting the solutes in at least one container to determine the type of the re-constituted solution; and/or
Selecting the volume of fluid that flows into the at least one container to determine the concentration of the re-constituted solution.

Furthermore, the method may include the step of connecting the bag to a dialysis flow path, such that the re-constituted solution may circulate in the dialysis flow path.

Also described herein is a system for dialysis wherein a preparator as described herein are fluidly connected to a dialysis machine of any type. The system may comprise further components.

Also described herein is a method of dialysis comprising the steps of:-
Fluidly connecting system comprising preparator of the current invention to a dialysis machine;
fluidly connecting said dialysis machine to a patient;
and drainage from the patient.

A method of dialysis may further comprise the step of fluidly connecting a dialysis system of the current invention to a dialysis machine to a patient.

A method of dialysis may comprise the steps of:-
Fluidly connecting a dialysis a machine to a patient via a catheter and transfer set;
Delivering dialysate from the machine to the patient, wherein said dialysate is reconstituted in the preparator using the method described above;
wherein said dialysate is warmed prior to delivery of this fluid to the patient;
wherein the dialysate fluid remains in the patient for a predetermined dwell time; and wherein said fluid is then removed from the patient.

The system may be is controlled by a controller.

Any method of dialysis described herein may comprise an initial priming step, wherein the dialysis machine is primed before connection to the patient.

The methods of dialysis described herein may be peritoneal dialysis, hemodialysis, hemofiltration, hemodiafiltration or ultrafiltration.

In preferred embodiments, the methods of dialysis described herein are suitable for peritoneal dialysis.

In preferred embodiments, the methods of dialysis described herein are suitable for dialysis in a home environment.

### Kit

A fourth aspect of the present invention provides a kit for use in a dialysis system.

Said kits may comprise a preparator and cycler, wherein said preparator comprises a purified water source fluidly connectable to at least one container containing one or more solutes for dissolution, at least one measuring device, a gyroscope configured to measure the orientation and/or angular velocity of the preparator or a component thereof and an accelerometer configured to measure linear acceleration of the preparator or a component thereof.

Said kits may further comprise a controller.

Said kits may comprise one or more nozzles. The nozzles may be Venturi nozzles.

Said kits may further comprise connectors and/or tubing/pipping and/or a drain.

Said kits may also comprise a water purification unit.

Said kits may also comprise a heater.

The at least one measuring device in said kits may be a scale or scale system.

Said kits may also include one or more tags or labels, such as smart tags, to enable identification of the parts and contents thereof

### Uses

The systems and kits of the invention may be used in dialysis, particularly hemodialysis peritoneal dialysis, hemofiltration, hemodiafiltration or ultrafiltration. Particularly they are used in dialysis carried out at home, or another environment away from a hospital or medical practice. They may also be used in a hospital or any kind of medical practice.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

1. A dialysis system comprising a preparator and cycler, wherein said preparator comprises a purified water source fluidly connectable to at least one container containing one or more solutes for dissolution, at least one measuring device, a gyroscope configured to measure the orientation and/or angular velocity of the preparator or a component thereof and an accelerometer configured to measure linear acceleration of the preparator or a component thereof.

2. A dialysis system according to claim 1, further comprising a controller.

3. A dialysis system according to any preceding claim, wherein if the gyroscope measures a change in the orientation and/or angular velocity of the preparator or a component thereof, it provides feedback which causes the at least one measuring device to be re-calibrated if the change in orientation and/or angular velocity of the preparator or a component thereof falls outside of a pre-determined range.

4. A dialysis system according to any preceding claim, wherein if the accelerometer measures a change in the linear acceleration of the preparator or a component thereof, it provides feedback which causes the at least one measuring device to be re-calibrated if the change in linear acceleration of the preparator or a component thereof falls outside of a pre-determined range.

5. A dialysis system according to any preceding claim wherein if the accelerometer measures a change in the linear acceleration of the preparator or a component thereof falling outside of a pre-determined range, and/or the gyroscope measures a change in the orientation and/or angular velocity of the preparator or a component thereof outside of a second pre-determined range the dialysis system is shut down due to incorrect use.

6. A dialysis system according to any preceding claim wherein the gyroscope and/or the accelerometer monitors orientation and/or angular velocity and/or linear acceleration of the preparator or a component thereof continuously during use.

7. A dialysis system according to any preceding claim, wherein the at least one measuring device comprises a scale or system of scales.

8. A dialysis system according to any preceding claim, wherein at least one hydraulic flow generator is provided in at least one of the fluid paths between the water source and the containers and the containers and the cycler.

9. A dialysis system according to any preceding claim peritoneal dialysis, hemodialysis, hemofiltration, hemodiafiltration or ultrafiltration.

10. A preparator for use in a dialysis system according to any of claims 1-9.

11. A method of re-constituting a solution comprising one or more solutes in a fluid comprising the steps of:-
flowing a fluid, preferably a purified water, through a preparator, said preparator comprising a at least one container fluidly connected to the purified water source, said container containing one or more solutes for dissolution, and said preparator further comprising at least one measuring device, a gyroscope and an accelerometer;
wherein the at least one measuring device is a scale or scale system;
wherein said scale or scale system measures the mass of the solutions created by the water flowing into the at least one container in order to create a dialysate comprising the correct concentration of solutes;
wherein the gyroscope is configured to measure the orientation and/or angular velocity of the preparator or a component thereof and the accelerometer is configured to measure linear acceleration of the preparator or a component thereof;
wherein if the gyroscope measures a change in the orientation and/or angular velocity of the preparator or a component thereof, it provides feedback which causes the at least one measuring device to be re-calibrated if the change in orientation and/or angular velocity of the preparator or a component thereof falls outside of a pre-determined range; and/or
wherein if the accelerometer measures a change in the linear acceleration of the preparator or a component thereof, it provides feedback which causes the at least one measuring device to be re-calibrated if the change in linear acceleration of the preparator or a component thereof falls outside of a pre-determined range; and/or
wherein if the accelerometer measures a change in the linear acceleration of the preparator or a component thereof falling outside of a pre-determined range, and/or the gyroscope measures a change in the orientation and/or angular velocity of the preparator or a component thereof outside of a second pre-determined range the dialysis system is shut down due to incorrect use.

12. A method of dialysis comprising the steps of:-
Fluidly connecting a dialysis a machine to a patient via a catheter and transfer set;
Delivering dialysate from the machine to the patient, wherein said dialysate is reconstituted in the preparator using the method of claim 11;
Warming the dialysate prior to delivery of this fluid to the patient;
wherein the dialysate fluid remains in the patient for a predetermined dwell time; and wherein said fluid is then removed from the patient.

13. The method according to claim 11 wherein the system may be is controlled by a controller.

14. Us of the method according to claim 12 or 13 for peritoneal dialysis.

15. A kit for use in a dialysis system, said kits comprising a preparator and cycler, wherein said preparator comprises a purified water source fluidly connectable to at least one container containing one or more solutes for dissolution, at least one measuring device, a gyroscope configured to measure the orientation and/or angular velocity of the preparator or a component thereof and an accelerometer configured to measure linear acceleration of the preparator or a component thereof, and optionally further comprising a controller.
